# EUROPÄISCHE PATENTSCHRIFT

(11) **EP 1 290 408 B1**
(45) Veröffentlichungstag und Bekanntmachung des Hinweises auf die Patenterteilung: **06.10.2004**
(21) Anmeldenummer: 01943440.6
(22) Anmeldetag: 23.05.2001
(51) Int. Cl.: G01C 11/06, G01C 15/02, A61B 5/107, A43D 1/02

(54) **VERFAHREN UND ANORDNUNGEN ZUR PHOTOGRAMMETRISCHEN ERFASSUNG DER RAUMFORM EINES OBJEKTS**
METHOD AND ASSEMBLY FOR THE PHOTOGRAMMETRIC DETECTION OF THE 3-D SHAPE OF AN OBJECT
PROCEDE ET DISPOSITIF PERMETTANT LA DETECTION PHOTOGRAMMETRIQUE DE LA FORME D'UN OBJET DANS L'ESPACE

(30) Priorität: 27.05.2000 DE 10025922
(43) Veröffentlichungstag der Anmeldung: 12.03.2003
(73) Patentinhaber: corpus.e AG, 70178 Stuttgart (DE)
(72) Erfinder: Massen, Robert, Prof. Dr., 78337 Öhningen-Wangen (DE)
(74) Vertreter: Degwert, Hartmut, Dipl.-Phys.
(86) Internationale Anmeldenummer: PCT/EP2001/005935
(87) Internationale Veröffentlichungsnummer: WO 2001/092824

(56) Entgegenhaltungen:
- EP-B- 0 760 622

## Beschreibung

Die Erfindung betrifft Verfahren und Anordnungen zur photogrammetrischen Erfassung der Raumform eines Objekts.

Die berührungslose Erfassung der 3D-Form von Körpern und Objekten wie Automobilkarosserien, menschlichen Körperteilen usw. ist eine wichtige Meßmethode bei der Qualitätskontrolle, dem Reversed Engineering, der Erzeugung von numerischen 3D-Modellen von physikalisch existierenden Körpern usw.

Es sind in der Literatur und auf dem Markt eine ganze Reihe von unterschiedlichen Verfahren bekannt, welche entweder auf der Streifenprojektion, der Laserlinienprojektion, der Laserlaufzeitmessung, der Photogrammetrie und teilweise auch auf kombinierten Verfahren beruhen.

Besonders kostengünstig sind die sogenannten passiven Verfahren der Nahbereichsphotogrammetrie, da hier lediglich kalibrierte Photoapparate oder Digitalkameras, aber keine teuren Projektionseinheiten und genau kalibrierte mechanische Aufbauten benötigt werden.

Ein typischer Vertreter dieser Gruppe ist die "Photomodeler"-Software der Firma EOS (siehe Internetseite www.photomodeler.com). Mit dieser Software ist es möglich, aus verschiedenen, sich, was die eingefangenen Objektteile angeht, überlappenden Aufnahmen eines Objektes dessen numerisches 3D-Modell zu entwickeln. Hierbei müssen allerdings manuell am Bildschirm die Positionen von auf den zu vermessenden Körper aufgeklebten Marken oder von markanten Punkten wie Ecken, Linien dieser Körper vom Benutzer in den verschiedenen 2D-Kameraaufnahmen angeklickt werden und paarweise "referenziert" werden, d.h. gleiche Marken müssen manuell in den verschiedenen Aufnahmen einander zugeordnet werden. Wird eine dichte Folge von XYZ-Koordinatenwerten benötigt, so sind mehrere hundert bis tausend solcher Zuordnungen manuell durchzuführen. Dies ist nicht nur extrem langwierig, sondern auch fehlerbehaftet, da die Zuordnung vieler gleichaussehender Marken fehlerfrei kaum noch möglich ist.

Es ist aus der Photogrammetrie auch bekannt, Marken mit codierter Punktnummer (codierte Punktmarken) zu verwenden, die ein Muster mit codierter Punktnummer tragen. Dazu werden linien-, ring- oder flächenhaft verteilte Punktcodierungen benutzt, die um den eigentlichen Meßpunkt herum angeordnet sind. Die Identität der codierten Punktmarken kann durch eine Bildverarbeitung automatisch erkannt werden, woraufhin dann die paarweise Zuordnung der Punkte in den Aufnahmen automatisch erfolgen kann.

Diese codierten Punktmarken müssen allerdings recht groß sein, um einen Code wie z.B. einen radialen Strichcode, einen 2-dimensionalen Punktcode o.ä. unterzubringen. Jede Marke benötigt in der Regel einen individuellen Code. Dies wiederum verhindert es, diese Methode bei kleinen Körpern mit vielen benötigten Marken anzuwenden, da diese Punktmarken wegen der engen Platzverhältnisse nicht mehr in genügender Zahl aufgebracht werden können.

Es sind auch photogrammetrische Verfahren bekannt, die ohne codierte Marken auskommen und iterativ die Zuordnung gleichaussehender Marken bestimmen. Diese Verfahren setzen aber genau kalibrierte Kameras voraus, sind sehr langwierig und verlangen vom Anwender eine gute Kenntnis der photogrammetrischen Randbedingungen, insbesondere der geeigneten Aufnahmepositionen und des erforderlichen Überlappungsgrades, damit die iterativen Lösungsverfahren konvergieren.

In der Patentschrift EP 0 760 622 "Verfahren und Anordnung zur dreidimensionalen Erfassung der Raumform von Körpern und Körperteilen", Erfinder und Anmelder Robert Massen, sind ein Verfahren und eine Anordnung beschrieben, mit welcher zu digitalisierende Körperteile durch Überstreifen eines elastischen, eng anliegenden Überzugs, welcher photogrammetrisch auswertbare Marken aufweist, einfach und kostengünstig markiert werden können. Beispielhaft wird in dieser Patentschrift die Markierung eines menschlichen Fusses zur Herstellung von 3D-Modellen für die Fertigung von Prothesen oder Maßschuhen erläutert. Mit diesem Verfahren ist es einfach und kostengünstig möglich, eine große Zahl von Marken aufzubringen und damit die Grundlage für die photogrammetrische Gewinnung von dichten XYZ-Koordinaten zu legen. Das Problem der einfachen automatischen Registrierung von Marken aus dem Überlappungsbereich der unterschiedlichen Aufnahmen ist damit aber noch nicht gelöst.

Die Aufgabe der vorliegenden Erfindung besteht darin, Verfahren und Anordnungen zur photogrammetrischen Erfassung der Raumform eines Objekts zu schaffen, die zur automatischen Referenzierung photogrammetrischer Marken und zur hochauflösenden Digitalisierung von Objekten, Körpern und Körperteilen geeignet sind und insbesondere die oben erwähnten Nachteile überwinden, die bei photogrammetrischen Verfahren auftreten, bei denen codierte Punktmarken verwendet werden.

Diese Aufgabe wird durch ein Verfahren zur photogrammetrischen Erfassung der Raumform eines Objekts gelöst, bei dem auf der Oberfläche des Objekts Punktmarken und eine oder mehrere Flächenmarken angebracht werden, die jeweils mehrere Punktmarken umfassen, einen Hintergrund der Punktmarken bilden und deren Fläche eine charakteristische optische Gestaltung aufweist, mehrere photogrammetrische Aufnahmen des Objekts aus jeweils verschiedenen Ansichten gemacht werden, eine Bildverarbeitung der Aufnahmen durchgeführt wird, bei der zunächst die sich in den Aufnahmen jeweils entsprechenden Flächenmarken anhand ihrer charakteristischen optischen Gestaltung einander zugeordnet werden und dann mithilfe der Flächenmarkenzuordnung die von den Flächenmarken umfaßten und sich in den Aufnahmen jeweils entsprechenden Punktmarken einander zugeordnet werden, und anhand der Punktmarkenzuordnung die Raumform des Objekts mit einem photogrammetrischen Auswerteverfahren ermittelt wird.

Darüber hinaus wird diese Aufgabe durch ein Verfahren zur photogrammetrischen Erfassung der Raumform eines Objekts gelöst, bei dem auf der Oberfläche des Objekts Punktmarken so angebracht werden, daß Gruppen aus mehreren Punktmarken gebildet werden, wobei die Punktmarken in einer Gruppe so zueinander angeordnet sind, daß sich eine charakteristische Punktmarkenanordnung ergibt, mehrere photogrammetrische Aufnahmen des Objekts aus jeweils verschiedenen Ansichten gemacht werden, eine Bildverarbeitung der Aufnahmen durchgeführt wird, bei der zunächst die sich in den Aufnahmen jeweils entsprechenden charakteristischen Punktmarkenanordnungen einander zugeordnet werden und dann mithilfe dieser Zuordnung die von den charakteristischen Punktmarkenanordnungen umfaßten und sich in den Aufnahmen jeweils entsprechenden Punktmarken einander zugeordnet werden, und anhand der Punktmarkenzuordnung die Raumform des Objekts mit einem photogrammetrischen Auswerteverfahren ermittelt wird.

Darüber hinaus wird die Aufgabe der Erfindung durch eine Anordnung zur photogrammetrischen Erfassung der Raumform eines Objekts mit einem Aufnahmesystem zur Erstellung photogrammetrischer Aufnahmen verschiedener Ansichten des Objekts und einem System zur Vermessung und Auswertung der Aufnahmen sowie zur Ermittlung der Raumform des Objekts gelöst, die dadurch gekennzeichnet ist, daß die Anordnung darüber hinaus auf der Oberfläche des Objekts angebrachte Punktmarken und eine oder mehrere Flächenmarken aufweist, die jeweils mehrere Punktmarken umfassen, einen Hintergrund der Punktmarken bilden und deren Fläche eine charakteristische optische Gestaltung aufweist.

Schließlich wird die erfindungsgemäße Aufgabe durch eine Anordnung zur photogrammetrischen Erfassung der Raumform eines Objekts mit einem Aufnahmesystem zur Erstellung photogrammetrischer Aufnahmen verschiedener Ansichten des Objekts und einem System zur Vermessung und Auswertung der Aufnahmen sowie zur Ermittlung der Raumform des Objekts gelöst, die dadurch gekennzeichnet ist, daß die Anordnung darüber hinaus Punktmarken aufweist, die so auf der Oberfläche des Objekts angebracht sind, daß Gruppen aus mehreren Punktmarken gebildet werden, wobei die Punktmarken in einer Gruppe so zueinander angeordnet sind, daß sich eine charakteristische Punktmarkenanordnung ergibt.

Durch die erfindungsgemäßen Verfahren und Anordnungen ergibt sich folgende vorteilhafte Wirkung:
Die Einbeziehung von charakterisch gestalteten Hintergründflächen hinter den Punktmarken zur Referenzierung bzw. die Zusammenfassung nichtcodierter Punktmarken zu charakteristischen übergeordneten Punktmarkenanordnungen ermöglicht auch dann eine bequeme und präzise automatische Referenzierung von Punkten, wenn das Objekt, dessen Raumform photogrammetrisch bestimmt werden soll, klein ist und/oder eine Vielzahl von Punktmarken für eine hinreichend Bestimmung der Raumform mittels photogrammetrischer Verfahren benötigt.

Vorteilhafte Weiterbildungen der Erfindung sind in den abhängigen Patentansprüchen gekennzeichnet.

Weitere Merkmale und Vorteile der Erfindung ergeben sich aus der folgenden Beschreibung eines Ausführungsbeispiels anhand der Zeichnungen. In den Zeichnungen zeigen:
Fig. 1 zwei verschiedene zum Stand der Technik gehörende codierte Punktmarken,
Fig. 2 eine Ausführungsform der Erfindung bei einem menschlichen Fuß, der mit einem Überzug überzogen ist, auf dem Punktmarken und farbige Flächenmarken angebracht sind,
Fig. 3, 4 und 5 schematische Darstellungen, die zeigen, wie die photogrammetrische Auswertung mithilfe der in der Fig. 2 dargestellten Punkt- und Flächenmarken erfolgt,
Fig. 6 eine weitere Ausführungsform der Erfindung, bei der die Punktmarken in Gruppen mit charakteristischen Punktmarkenanordnungen in Form eines Sternbildes zusammengefaßt sind,
Fig. 7 eine schematische Darstellung von Punktmarken wie sie bei einer bevorzugten Ausführungsform der Erfindung verwendet werden.

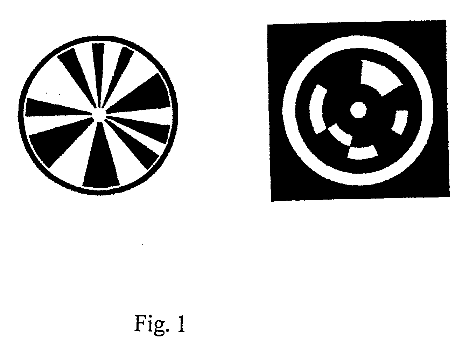

Fig. 1 zeigt zwei mögliche Ausführungsformen von den in der Beschreibungseinleitung erwähnten und zum Stand der Technik gehörenden codierten Punktmarken.

Die Erfindung wird nun beispielhaft, aber nicht einschränkend am Anwendungsfall der optischen Erfassung der Raumform eines menschlichen Fusses zur Gewinnung der 3D-Daten, welche für eine automatische Auswahl passender Schuhformen oder Schuhleistenformen benötigt werden, erklärt.

Hierbei wird ebenfalls beispielhaft aber nicht einschränkend davon ausgegangen, daß der Fuß gemäß dem oben genannten Patent EP 0 760 622 mit einem eng anliegenden elastischen Überzug bekleidet ist, welcher auf seiner Oberfläche gleichaussehende, nicht codierte Punktmarken in Form kleiner Kreuze aufweist.

Beispielhaft wird die einer bevorzugten Ausführungsform der Erfindung zugrunde liegende Idee der Hintergrundmarkierung bei einer regionenweise durchgeführten Farbgebung des Hintergrundes der Punktmarken erläutert. Aus Gründen des nicht möglichen Abdrucks von Farbbildern in Patentschriften wird die unterschiedliche Farbgebung durch unterschiedliche Schwarz/weiß-Texturen in den Figuren dargestellt.

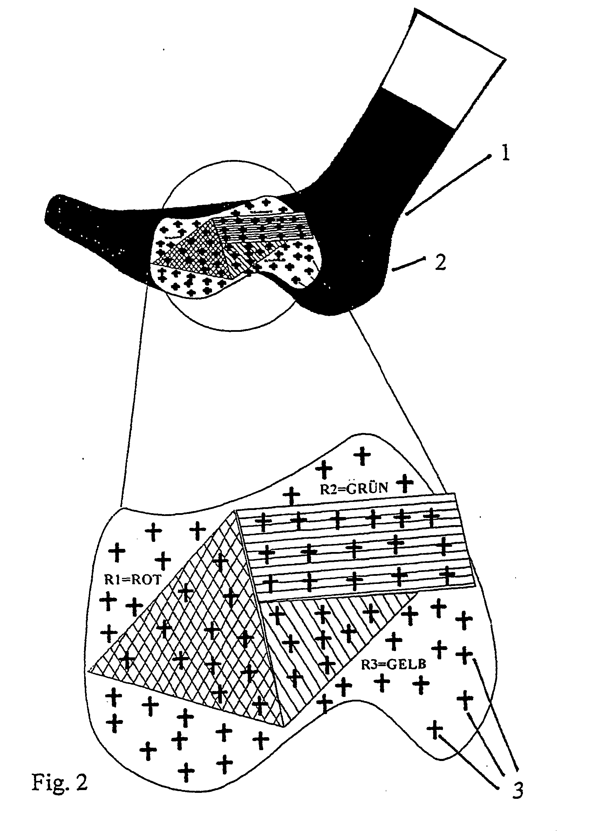

In Fig. 2 ist ein menschlicher Fuß 1 dargestellt, der mit einem eng anliegenden elastischen Überzug 2 bekleidet ist. Der Überzug 2 weist auf seiner Oberfläche eine große Zahl identisch aussehender photogrammetrischer Punktmarken 3 in Form kleiner Kreuze auf.

Der Kreuzungspunkt der beiden Linien der Punktmarken stellt eine mit Verfahren der zweidimensionalen Bildverarbeitung bestimmbare XY-Koordinate in der jeweils betrachteten Aufnahme dar, welche auch bei unterschiedlichen Aufnahmewinkeln, perspektivischen Verzerrungen usw. genau als zweidimensionale Koordinate bezüglich des Koordinatensystems der jeweiligen Aufnahme bestimmt werden kann. Werden zur Aufnahme z.B. digitale Kameras benutzt, so ist das jeder Aufnahme zugrunde liegende Koordinatensystem durch die Zeilen- und Spaltenstruktur der Sensormatrix sowie durch den Durchstoßpunkt der optischen Achse bestimmt.

Beispielhaft sind in der Fig. 1 nur drei Regionen R1, R2 und R3 mit den unterschiedlichen Hintergrundfarben ROT, GRÜN und GELB wiedergegeben. Es ist dem Fachmann der Bildverarbeitung bekannt, daß mit Farbkameras solche Farbregionen anhand der auftretenden, vorher dem System eintrainierten Farben automatisch segmentiert, d.h. automatisch erkannt und voneinander unterschieden werden können. Solche Verfahren sind z.B. in der Patentanmeldung DE 36 39 636 C2 "Automatische Inspektion von Textilbahnen", Erfinder und Anmelder Robert Massen, beschrieben.

Es ist außerdem bekannt, daß eine solche Farbsegmentierung besonders robust und unabhängig vom Betrachtungswinkel erfolgen kann, wenn man Farbklassifikatoren einsetzt, welche unabhängig von der (vom Betrachtungswinkel abhängigen) Helligkeit sind. Dies kann z.B. durch Klassifikation jedes Bildpunktes anhand des Farbtons nach der Konvertierung der,RGB-Farbsignale in den HSI-Farbraum ( Hue, Saturation, Intensity) geschehen.

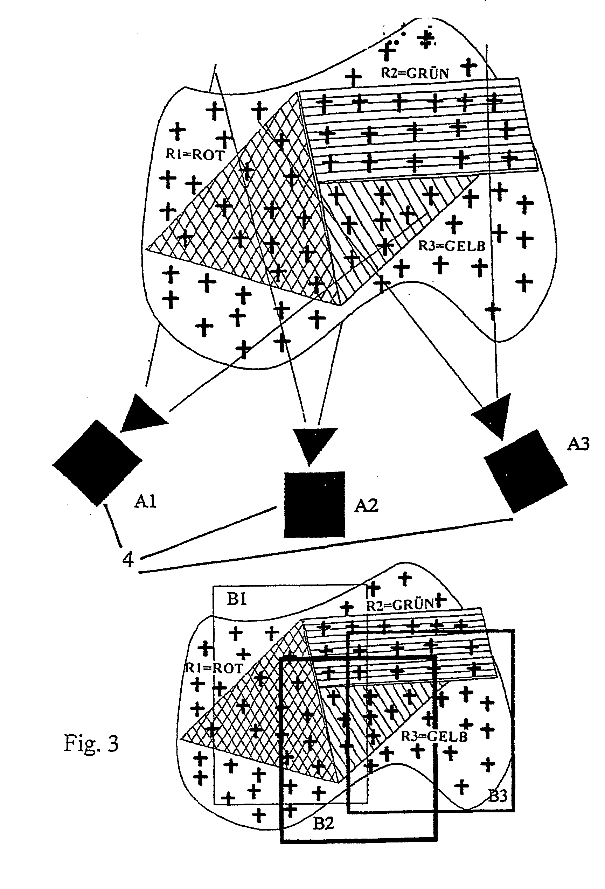

Wie in Fig. 3 verdeutlicht, werde beispielhaft die durch die drei Regionen R1, R2 und R3 definierte Oberfläche des zu digitalisierenden Körpers aus drei unterschiedlichen Aufnahmepositionen A1, A2 und A3 mit einer Digitalkamera 4 aufgenommen. Die entsprechenden drei zweidimensionalen Bilder B1, B2 und B3 zeigen jeweils sich überlappende Ausschnitte der Körperoberfläche.

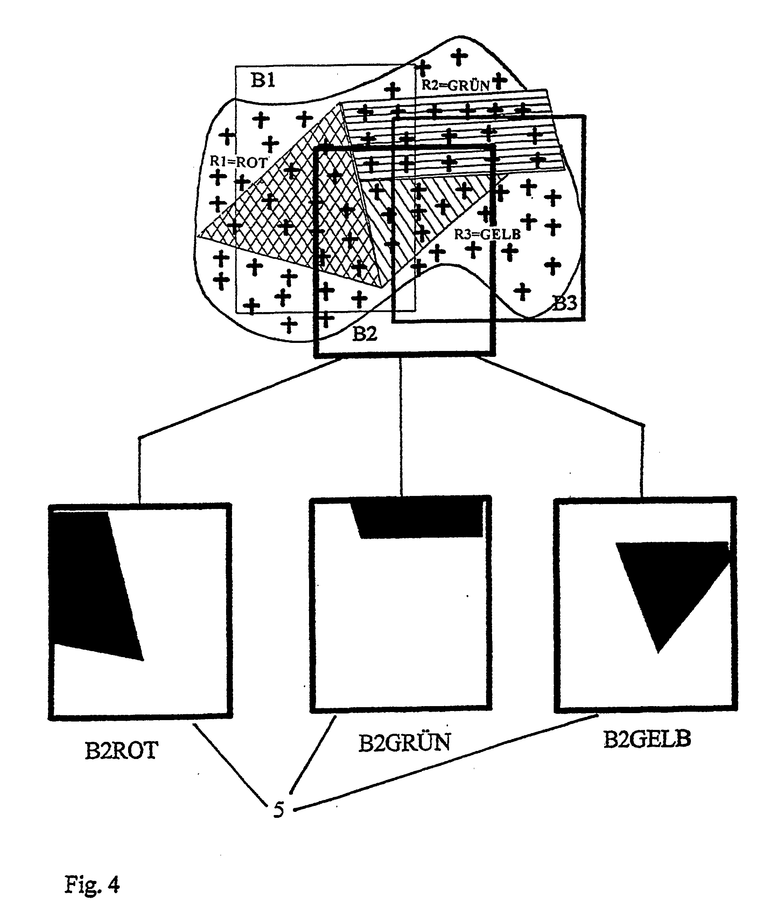

Durch die bildpunktweise durchgeführte Farbklassifikation aller drei Bilder in die Farben ROT, GRÜN und GELB kann jedes der drei Bilder B1, B2 und B3 in drei sog. Farbklassenbilder 5 (in der Fig. 4)
B1-> B1ROT,B1GRÜN,B1GELB
B2-> B2ROT,B2GRÜN,B2GELB
B3-> B3ROT,B3GRÜN,B3GELB
, wie in Fig. 4 am Beispiel der Region B2 gezeigt, automatisch zerlegt werden. Damit ist aber bereits eine automatische Zuordnung aller paarweise zueinander passenden Regionen erfolgt, d.h. es sind noch nicht die photogrammetrischen Marken, aber bereits die Hintergrundregionen R1, R2 und R3 dieser Marken referenziert.

Die Hintergrundregionen, die jeweils mehrere Punktmarken umfassen, werden hier im folgenden wegen ihrer flächigen Gestaltung in Analogie zu den bekannten Punktmarken auch als Flächenmarken bezeichnet.

Erfindungsgemäß werden die Punktmarken innerhalb von zwei referenzierten Regionen (Flächenmarken) durch Vergleich ihrer Lage bezüglich der Regionengrenzen und/oder durch Auswertung der von mehreren Punktmarken gebildeten unterscheidbaren Figuren und/oder durch leichte Veränderungen ihrer Form einander automatisch zugeordnet.

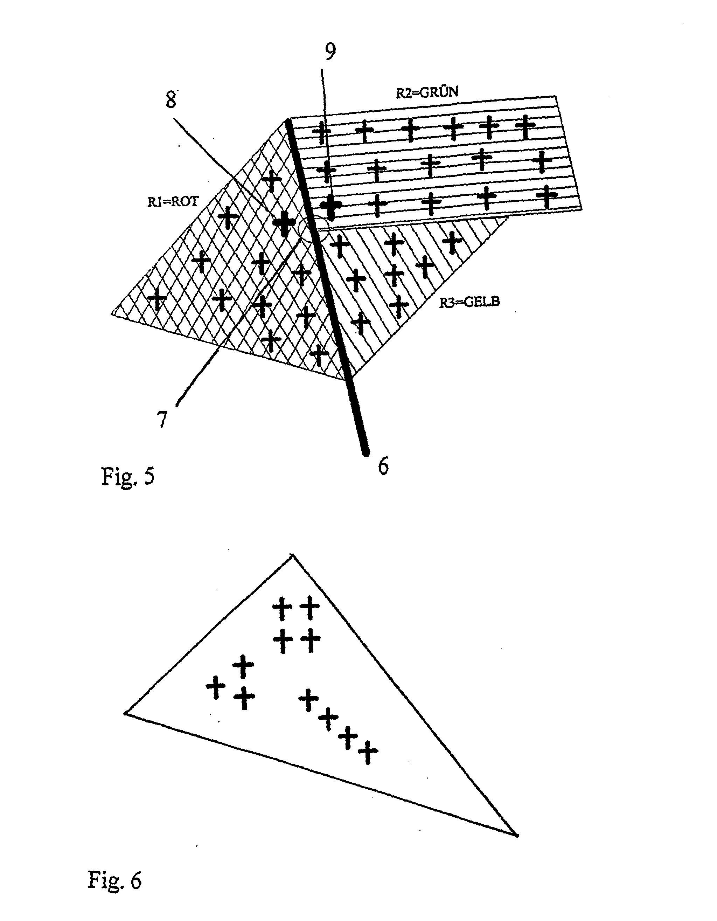

Fig. 5 zeigt beispielhaft, wie aus den in den Farbklassenbildern enthaltenen Informationen über die Regionengrenzen und den von Regionen gebildeten Ecken die unmittelbar an dieser Grenze liegenden photogrammetrischen Punktmarken einander zugeordnet werden können.

Beispielhaft sei die dreieckförmige rote Region R1 entlang einer Seite benachbart zu der ebenfalls dreieckförmigen gelben Region R3 und zu der grünen Region R2, welche die Form eines Parallelogramms aufweist.

Entlang der betrachteten Seitenkante 6 bildet sich außerdem eine "Dreiländergrenze" aus, d.h. ein markanter Punkt 7, in dem drei farblich unterschiedlich markierte Flächenmarken (Regionen) aufeinander stoßen. Dieser Punkt kann leicht aus dem Vergleich aller Farbklassenbilder mit einem Nachbarschaftsoperator in den beiden sich überlappenden Aufnahmen B1 und B2 gefunden werden. Solche Verfahren sind dem Fachmann der Farbbildverarbeitung bekannt. Liegt die Koordinate dieses Punktes in den zwei betrachteten Bildern B1 und B2 fest, so können leicht mit klassischen Suchverfahren beginnend an diesem Startpunkt, parallel zur Farbregionengrenze und im Inneren der korrespondierenden Regionen die nichtcodierten photogrammetrischen Punktmarken, hier beispielhaft die kleinen s/w Kreuze 8 und 9 einander zugeordnet werden.

Ein weiterer Vorteil der farbcodierten Regionen ist die Möglichkeit, automatisch nach jeder Bildaufnahme zu prüfen, ob der Überlappungsgrad zweier Aufnahmen ausreichend groß ist. Der Überlappungsgrad ergibt sich aus der Anzahl farbidentischer Bildpunkte in jeder Aufnahme.

Neben der flächenmäßigen Markierung der Hintergrundregionen durch Farbe, Textur und ähnliche Merkmale kann auch die Form der Hintergrundregion mit Informationen beaufschlagt werden, welche die automatische Referenzierung erleichtern. So sind z.B. dreieckförmige Regionen geeignet, anhand der spitzen Ecken leicht mit Verfahren der 2D-Bildverarbeitung erkannt und zugeordnet zu werden.

Neben der automatischen Referenzierung der photogrammetrischen Punktmarken anhand von deren Lage bezüglich der Regionengrenzen können auch andere Merkmale dieser nichtcodierten Punktmarken verwendet werden. So werden z.B., wie in Fig. 6 gezeigt, erfindungsgemäß die photogrammetrischen Punktmarken zu charakteristischen Punktmarkenanordnungen in der Art von "Sternbildern" gruppiert, d.h. die relative Lage mehrerer nichtcodierter Punktmarken innerhalb einer Region wird so gewählt, daß sich automatisch identifizierbare geometrische Formen bilden wie Dreiecke, Quader, längliche Strukturen etc.. Insbesondere sind dabei solche Strukturen vorteilhaft, welche invariant gegenüber affinen Bildtransformationen wie Translation, Rotation, Dehnung, Streckung, perspektivische Verzerrung sind. Diese Bildverzerrungen entstehen immer, wenn ein Bildausschnitt aus zwei verschiedenen Aufnahmepositionen aufgenommen wird. Solche Gruppierungsformen sind immer möglich, da die Position der photogrammetrischen Marken insbesondere bei Körpern mit stetig gekrümmten Oberflächen in Grenzen frei gewählt werden kann.

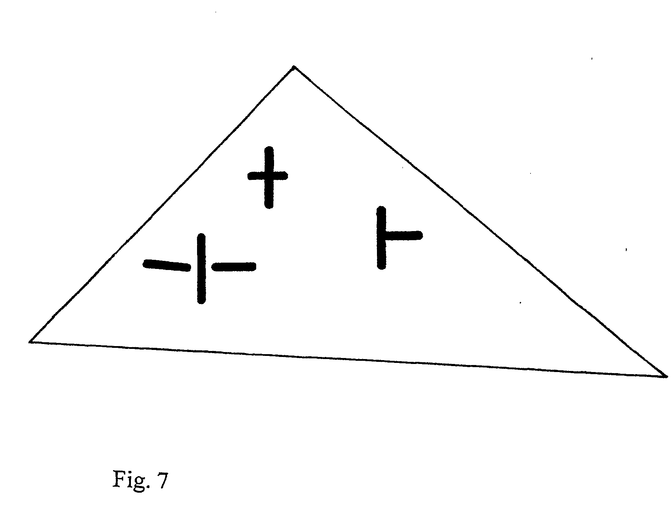

Auch können die photogrammetrischen Punktmarken innerhalb einer Region durch leichte Veränderungen ihrer Form identifizierbar gemacht werden. Dies kann wie in Fig. 7 z.B. durch Variation der Strichlänge und des Strichbildes geschehen. Diese Art der Codierung unterscheidet sich wesentlich von den bekannten Verfahren der Codierung durch Strichcodes o.ä. In dem erfindungsgemäßen Verfahren werden nur die Punktmarken innerhalb einer Region unterschiedlich markiert; es brauchen nicht alle Punktmarken, welche den gesamten Körper bedecken, identifizierbar sein. Die Codierung kann daher sehr einfach ausfallen und braucht nur einige wenige Markenmuster zu unterscheiden. Daher können kleine Markierungen verwendet werden.

Durch die Kombination der einzelnen Verfahren der Referenzierung der photogrammetrischen Punktmarken innerhalb einer Flächenmarke (Region) können auch bei größeren Farbregionen aus zwei oder mehreren sich überlappenden Aufnahmen die in gleichen Regionen enthaltenen Marken leicht mit Verfahren der zweidimensionalen Bildverarbeitung und Mustererkennung einander zugeordnet werden.

Damit ist über die beiden Verfahrensschritte:
a) Referenzierung einander zugeordneter Hintergrundregionen anhand flächiger Merkmale wie Farbe, Textur, Polarisationsgrad, NIR-Reflexion, Polarisationsgrad u.ä.
b) Referenzierung der nicht codierten photogrammetrischen Punktmarken innerhalb einer Region anhand ihrer Lage bezüglich der Regionen, ihrer Gruppierungsstrukturen und einfacher Formmerkmale
eine vollständige automatische photogrammetrische Auswertung eines erfindungsgemäß markierten Körpers oder Körperteils möglich.

Selbstverständlich ist es erfindungsgemäß auch möglich, das beschriebene Verfahren mit traditionell codierten photogrammetrischen Punktmarken zu kombinieren, wie sie z.B. in der Fig. 1 dargestellt sind.

Bei der Ausführungsform der Erfindung mit den Flächenmarken sind verschiedene Abwandlungen denkbar. Es kommt stets nur darauf an, daß die Flächenmarken eine bestimmte optische Gestaltung aufweisen, so daß bei der Bildverarbeitung unterschiedliche Flächenmarken voneinander unterschieden werden können. Diese optische Gestaltung kann sich dabei z.B. in einer charakteristischen Form, einer charakteristischen Farbe, einem charakteristischen Grauwert, einer charakteristischen Textur, d.h. einem charakteristischen Muster oder einer charakteristischen Struktur, einem charakteristischen Glanz, einer auf der Fläche der Flächenmarken aufgebrachten Oberflächenbeschichtung mit charakteristischem Polarisationsgrad oder einer charakteristischen Kombination der aufgezählten optischen Gestaltungsmerkmale bestehen. Die Flächenmarken können auch dadurch in ihrer optischen Gestaltung charakterisiert werden, daß sie entweder im sichtbaren oder im unsichtbaren Teil des Spektrums Strahlung aussenden.

Die Flächenmarken brauchen auch nicht notwendigerweise auf einem Überzug angebracht zu werden. Sie können ebensogut auch direkt auf dem Objekt angebracht werden. Darüber hinaus können z.B. auch selbstklebende Folien auf das Objekt geklebt werden, wobei auf den Folien die Punkt- und Flächenmarken aufgebracht sind. Schließlich ist es auch denkbar, daß die Punkt- und Flächenmarken auf die Oberfläche des Objekts projiziert werden. Das gleiche gilt natürlich für die Punktmarken bei der Ausführungsform mit den sternbildartigen Punktmarkenanordnungen.

Auch die Punktmarken können unterschiedliche Formen aufweisen, wobei sie nicht notwendigerweise aus Kreuzen bestehen müssen. Sie können z.B. auch aus den Schnittpunkten eines Netzes bestehen.

## Patentansprüche

1. Verfahren zur photogrammetrischen Erfassung der Raumform eines Objekts (1), **dadurch gekennzeichnet, daß**
auf der Oberfläche des Objekts (1) Punktmarken (3) und eine oder mehrere Flächenmarken (R1, R2, R3) angebracht werden, die jeweils mehrere Punktmarken (3) umfassen, einen Hintergrund der Punktmarken (3) bilden und deren Fläche eine charakteristische optische Gestaltung aufweist,
mehrere photogrammetrische Aufnahmen des Objekts aus jeweils verschiedenen Ansichten gemacht werden,
eine Bildverarbeitung der Aufnahmen durchgeführt wird, bei der zunächst die sich in den Aufnahmen jeweils entsprechenden Flächenmarken (R1, R2, R3) anhand ihrer charakteristischen optischen Gestaltung einander zugeordnet werden und dann mithilfe der Flächenmarkenzuordnung die von den Flächenmarken (R1, R2, R3) umfaßten und sich in den Aufnahmen jeweils entsprechenden Punktmarken (3) einander zugeordnet werden, und
anhand der Punktmarkenzuordnung die Raumform des Objekts (1) mit einem photogrammetrischen Auswerteverfahren ermittelt wird.

2. Verfahren nach Anspruch 1, bei dem die charakteristische optische Gestaltung der Fläche der Flächenmarken in einer charakteristischen Form, einer charakteristischen Farbe, einem charakteristischen Grauwert, einer charakteristischen Textur, d.h. einem charakteristischen Muster oder einer charakteristischen Struktur, einem charakteristischen Glanz, einer auf der Fläche der Flächenmarken aufgebrachten Oberflächenbeschichtung mit charakteristischem Polarisationsgrad oder einer charakteristischen Kombination der aufgezählten optischen Gestaltungsmerkmale besteht.

3. Verfahren nach einem der vorhergehenden Ansprüche, bei dem die Flächenmarken dadurch in ihrer optischen Gestaltung charakterisiert werden, daß sie entweder im sichtbaren oder im unsichtbaren Teil des Spektrums Strahlung aussenden.

4. Verfahren nach einem der vorhergehenden Ansprüche, bei dem das Anbringen der Punkt- und Flächenmarken auf dem Objekt in der Weise erfolgt, daß ein elastischer Überzug, auf dem die Punkt- und Flächenmarken aufgebracht sind, über das Objekt gezogen wird.

5. Verfahren nach einem der Ansprüche 1 bis 3, bei dem das Anbringen der Punkt- und Flächenmarken auf dem Objekt in der Weise erfolgt, daß auf die Oberfläche des Objekts selbstklebende Folien geklebt werden, auf denen die Punkt- und Flächenmarken aufgebracht sind.

6. Verfahren nach einem der Ansprüche 1 bis 3, bei dem das Anbringen der Punkt- und Flächenmarken auf dem Objekt in der Weise erfolgt, daß auf die Oberfläche des Objekts die Punkt- und Flächenmarken projiziert werden.

7. Verfahren nach einem der vorhergehenden Ansprüche, bei dem die von einer Flächenmarke umfaßten Punktmarken in einer bestimmten Anordnung zueinander und zu der Umfangslinie der Fläche der Flächenmarke angebracht werden.

8. Verfahren nach Anspruch 7, bei dem die Punktmarkenzuordnung mithilfe der Flächenmarkenzuordnung in der Weise erfolgt, daß die in verschiedenen Aufnahmen auftretenden Punktmarken anhand ihrer geometrischen Lage zu den Umfangslinien der von den Flächenmarken gebildeten Flächen und/oder anhand von von den Punktmarken innhalb der Flächenmarken gebildeten charakteristischen Punktmarkenanordnungen einander zugeordnet werden.

9. Verfahren zur photogrammetrischen Erfassung der Raumform eines Objekts, **dadurch gekennzeichnet, daß**
auf der Oberfläche des Objekts Punktmarken so angebracht werden, daß Gruppen aus mehreren Punktmarken gebildet werden, wobei die Punktmarken in einer Gruppe so zueinander angeordnet sind, daß sich eine charakteristische Punktmarkenanordnung ergibt,
mehrere photogrammetrische Aufnahmen des Objekts aus jeweils verschiedenen Ansichten gemacht werden,
eine Bildverarbeitung der Aufnahmen durchgeführt wird, bei der zunächst die sich in den Aufnahmen jeweils entsprechenden charakteristischen Punktmarkenanordnungen einander zugeordnet werden und dann mithilfe dieser Zuordnung die von den charakteristischen Punktmarkenanordnungen umfaßten und sich in den Aufnahmen jeweils entsprechenden Punktmarken einander zugeordnet werden, und
anhand der Punktmarkenzuordnung die Raumform des Objekts mit einem photogrammetrischen Auswerteverfahren ermittelt wird.

10. Verfahren nach Anspruch 9, bei dem das Anbringen der Punktmarken auf dem Objekt in der Weise erfolgt, daß ein elastischer Überzug, auf dem die Punktmarken aufgebracht sind, über das Objekt gezogen wird.

11. Verfahren nach Anspruch 9, bei dem das Anbringen der Punktmarken auf dem Objekt in der Weise erfolgt, daß auf die Oberfläche des Objekts selbstklebende Folien geklebt werden, auf denen die Punktmarken aufgebracht sind.

12. Verfahren nach Anspruch 9, bei dem das Anbringen der Punktmarken auf dem Objekt in der Weise erfolgt, daß diese auf die Oberfläche des Objekts projiziert werden.

13. Verfahren nach einem der vorhergehenden Ansprüche, bei dem die Punktmarken nichtcodiert sind.

14. Verfahren nach einem der vorhergehenden Ansprüche, bei dem die Punktmarken aus Kreuzen bestehen.

15. Verfahren nach einem der Ansprüche 1 bis 13, bei dem die Punktmarken aus den Schnittpunkten eines Netzes bestehen.

16. Anordnung zur photogrammetrischen Erfassung der Raumform eines Objekts mit einem Aufnahmesystem zur Erstellung photogrammetrischer Aufnahmen verschiedener Ansichten des Objekts und einem System zur Vermessung und Auswertung der Aufnahmen sowie zur Ermittlung der Raumform des Objekts, **dadurch gekennzeichnet, daß** die Anordnung darüber hinaus auf der Oberfläche des Objekts angebrachte Punktmarken und eine oder mehrere Flächenmarken aufweist, die jeweils mehrere Punktmarken umfassen, einen Hintergrund der Punktmarken bilden und deren Fläche eine charakteristische optische Gestaltung aufweist.

17. Anordnung nach Anspruch 16, die darüber hinaus einen elastischen Überzug aufweist, auf dem die Punktmarken und die eine Flächenmarke oder die mehreren Flächenmarken angebracht sind und der so ausgebildet ist, daß er über das Objekt gezogen werden kann und sich dabei an die Form des Objekts anpaßt.

18. Anordnung nach Anspruch 16, die darüber hinaus eine oder mehrere selbstklebende Folien aufweist, auf denen die Punktmarken und die eine Flächenmarke oder die mehreren Flächenmarken angebracht sind und die so ausgebildet sind, daß sie, wenn sie auf die Oberfläche des Objekts geklebt sind, eng an dem Objekt anliegen.

19. Anordnung nach Anspruch 16, die darüber hinaus ein Projektionssystem umfaßt, das so ausgebildet ist, daß damit die Punktmarken und die eine Flächenmarke oder die mehrere Flächenmarken auf das Objekt projiziert werden können.

20. Anordnung zur photogrammetrischen Erfassung der Raumform eines Objekts mit einem Aufnahmesystem zur Erstellung photogrammetrischer Aufnahmen verschiedener Ansichten des Objekts und einem System zur Vermessung und Auswertung der Aufnahmen sowie zur Ermittlung der Raumform des Objekts, **dadurch gekennzeichnet, daß** die Anordnung darüber hinaus Punktmarken aufweist, die so auf der Oberfläche des Objekts angebracht sind, daß Gruppen aus mehreren Punktmarken gebildet werden, wobei die Punktmarken in einer Gruppe so zueinander angeordnet sind, daß sich eine charakteristische Punktmarkenanordnung ergibt.

21. Anordnung nach Anspruch 20, die darüber hinaus einen elastischen Überzug aufweist, auf dem die Punktmarken angebracht sind und der so ausgebildet ist, daß er über das Objekt gezogen werden kann und sich dabei an die Form des Objekts anpaßt.

22. Anordnung nach Anspruch 20, die darüber hinaus eine oder mehrere selbstklebende Folien aufweist, auf denen die Punktmarken angebracht sind und die so ausgebildet sind, daß sie, wenn sie auf die Oberfläche des Objekts geklebt sind, eng an dem Objekt anliegen.

23. Anordnung nach Anspruch 20, die darüber hinaus ein Projektionssystem umfaßt, das so ausgebildet ist, daß damit die Punktmarken auf das Objekt projiziert werden können.

24. Verfahren oder Anordnung nach einem der vorhergehenden Ansprüche, bei dem bzw. der das Objekt ein Körperteil eines Menschen ist.

## Claims

1. A method for photogrammetric sensing the three-dimensional shape of an object (1) **characterized by**:
applying to the surface of said object (1) point marks (3) and one or more area marks (R1, R2, R3) each including a plurality of point marks (3), forming a background for said point marks (3) and the area of which is configured optically **characterized,**
taking a plurality of photogrammetic images of said object each in a different view,
processing the images in firstly assigning said area marks (R1, R2, R3) corresponding to each other in said images on the basis of their characteristic optical configuration and then
assigning the corresponding point marks (3) included in said area marks (R1, R2, R3) to each other with the aid of the area mark assignment, and
determining the three-dimensional shape of said object (1) on the basis of said point mark assignment in a photogrammetric analysis method.

2. The method as set forth in claim 1 wherein the characteristic optical configuration of said surface area of said area marks comprises a characteristic shape, a characteristic color, a characteristic gray value, a characteristic texture, i.e. a characteristic pattern or a characteristic structure, a characteristic shine, a surface coating applied to the surface area of said area marks having a characteristic degree of polarization or a characteristic combination of said cited optical configuration features.

3. The method as set forth in any of the preceding claims wherein said area marks are **characterized in** their optical configuration by emitting radiation in either the visible or invisible range of the spectrum.

4. The method as set forth in any of the preceding claims wherein applying said point marks and area marks to said object is done by pulling over said object an elastic envelope incorporating said point marks and area marks.

5. The method as set forth in any of the claims 1 to 3 wherein applying said point marks and area marks to said object is done by bonding to the surface of said object self-adhesive films incorporating said point marks and area marks.

6. The method as set forth in any of the claims 1 to 3 wherein applying said point marks and area marks to said object is done by projecting said point marks and area marks onto the surface of said object.

7. The method as set forth in any of the preceding claims wherein said point marks included in an area mark are applied in a specific arrangement to each other and to the peripheral line of the surface area of said area mark.

8. The method as set forth in claim 7 wherein said point mark assignment is done with the aid of the area mark assignment such that point marks materializing in the various images are assigned to each other by way of their geometric location to the peripheral lines of said surface areas formed by said area marks and/or by way of characteristic point mark arrangements formed by said point marks within said area marks.

9. A method for photogrammetric sensing the three-dimensional shape of an object **characterized by**:
applying point marks to the surface of said object so that groups of several point marks are formed, said point marks in a group being assigned to each other so that a characteristic point mark arrangement materializes,
taking a plurality of photogrammetic images of said object each in a different view,
processing the images in firstly assigning the corresponding characteristic point mark arrangements to each other and then
with the aid of this assignment assigning to each other said point marks included in said characteristic point mark arrangements corresponding each in said images, and
determining the three-dimensional shape of said object on the basis of point mark assignment in a photogrammetric analysis method.

10. The method as set forth in claim 9 wherein applying said point marks to said object is done by pulling over said object an elastic envelope incorporating said point marks.

11. The method as set forth in claim 9 wherein applying said point marks to said object is done by bonding to the surface of said object self-adhesive films incorporating said point marks.

12. The method as set forth in claim 9 wherein applying said point marks to said object is done by projecting them onto the surface of said object.

13. The method as set forth in any of the preceding claims wherein said point marks are non-coded.

14. The method as set forth in any of the preceding claims wherein each point mark is a cross.

15. The method as set forth in any of the claims 1 to 13 wherein each point mark is an intersection of a network.

16. An assembly for photogrammetric sensing the three-dimensional shape of an object comprising an imaging system for producing photogrammetric images in various views of said object and a system for gaging and analyzing the images as well as for determining the three-dimensional shape of said object, **characterized in that** said assembly further comprises point marks and one or more area marks applied to the surface of said object, said area marks each including a plurality of point marks, forming a background for said point marks, and the area of which is configured characteristically optical.

17. The assembly as set forth in claim 16 comprising in addition an elastic envelope incorporating said point marks and one or more area marks structured so that it can be pulled over said object in conforming to the shape of said object.

18. The assembly as set forth in claim 16 comprising in addition one or more self-adhesive films incorporating said point marks and one or more area marks structured so that when bonded to said object the films conform to the shape of said object.

19. The assembly as set forth in claim 16 comprising in addition a projecting system sturctured so that said point marks and one or more area marks can be projected onto said object.

20. An assembly for photogrammetric sensing the three-dimensional shape of an object comprising an imaging system for producing photogrammetric images in various views of said object and a system for gaging and analyzing the images as well as for determining the three-dimensional shape of said object, **characterized in that** said assembly further comprises point marks applied to the surface of said object so that groups of a plurality of point marks are formed, said point marks in a group being assigned to each other so that a characteristic point mark arrangement materializes.

21. The assembly as set forth in claim 20 comprising in addition an elastic envelope incorporating said point marks structured so that it can be pulled over said object in conforming to the shape of said object.

22. The assembly as set forth in claim 20 comprising in addition one or more self-adhesive films incorporating said point marks structured so that when bonded to said object the films conform to the shape of said object.

23. The assembly as set forth in claim 20 comprising in addition a projecting system sturctured so that said point marks can be projected onto said object.

24. The method or assembly as set forth in any of the preceding claims wherein said object is part of the human body.

## Revendications

1. Procédé de détermination photogrammétrique de la forme spatiale d'un objet (1), **caractérisé en ce que** :
sur la surface de l'objet (1) sont appliquées des marques ponctuelles (3) et une ou plusieurs marques superficielles (R1, R2, R3), qui comprennent chacune plusieurs marques ponctuelles (3) et qui constituent l'arrière-plan des marques ponctuelles (3) et dont la surface présente un aspect optique caractéristique ;
plusieurs prises de vues photogrammétriques de l'objet sont effectuées chacune avec une vue différente ;
un traitement d'image des prises de vues est réalisé, dans lequel les marques superficielles (R1, R2, R3) qui correspondent les unes aux autres dans les prises de vues sont d'abord associées les unes aux autres à l'aide de leur aspect optique caractéristique puis les marques ponctuelles (3) comprises dans les marques superficielles (R1,R2,R3), et qui correspondent les unes aux autres dans les prises de vues, sont associées les unes aux autres grâce à l'association des marques superficielles ; et
la forme spatiale de l'objet (1) est déterminée, à partir de l'association des marques ponctuelles, à l'aide d'un procédé d'analyse photogrammétrique.

2. Procédé selon la revendication 1, dans lequel l'aspect optique caractéristique de la surface des marques superficielles est constitué d'une forme caractéristique, d'une couleur caractéristique, d'un niveau de gris caractéristique, d'une texture caractéristique, c'est à dire d'un motif ou d'une structure caractéristique, d'une brillance caractéristique, d'un traitement de surface appliqué à la surface des marques superficielles avec un degré de polarisation caractéristique ou d'une combinaison caractéristique des attributs d'aspect optique énumérés ci-dessus.

3. Procédé selon l'une des revendications précédentes, dans lequel les marques superficielles sont caractérisées, en ce qui concerne leur aspect optique, par l'émission d'un rayonnement soit dans la partie invisible soit dans la partie visible du spectre.

4. Procédé selon l'une des revendications précédentes, dans lequel l'application des marques ponctuelles ou superficielles sur l'objet est réalisée de telle sorte qu'une enveloppe élastique, sur laquelle les marques ponctuelles et superficielles sont appliquées, soit enfilée sur l'objet.

5. Procédé selon l'une des revendications 1 à 3, dans lequel l'application des marques ponctuelles ou superficielles sur l'objet est réalisée de telle sorte que des films autocollants, sur lesquels les marques ponctuelles et superficielles sont appliquées, soient collés sur la surface de l'objet.

6. Procédé selon l'une des revendications 1 à 3, dans lequel l'application des marques ponctuelles ou superficielles sur l'objet est réalisée de telle sorte que les marques ponctuelles et superficielles soient projetées sur la surface de l'objet.

7. Procédé selon l'une des revendications précédentes, dans lequel les marques ponctuelles comprises dans une marque superficielle sont appliquées avec une disposition déterminée les unes par rapport aux autres et par rapport à la périphérie de la surface des marques superficielles.

8. Procédé selon la revendication 7, dans lequel l'association des marques ponctuelles est réalisée à l'aide de l'association des marques superficielles, de telle sorte que les marques ponctuelles qui apparaissent dans les différentes prises de vues soient associées les unes aux autres à l'aide de leur position géométrique par rapport aux périphéries des surfaces délimitées par les marques superficielles et/ou à l'aide des dispositions caractéristiques des marques ponctuelles à l'intérieur des marques superficielles.

9. Procédé de détermination photogrammétrique de la forme spatiale d'un objet, **caractérisé en ce que** :
sur la surface de l'objet, des marques superficielles sont appliquées de telle sorte que des groupes de plusieurs marques superficielles soient constitués, moyennant quoi les marques ponctuelles sont disposées dans un groupe les unes par rapport aux autres de telle sorte qu'une disposition caractéristique des marques ponctuelles soit obtenue ;
plusieurs prises de vues photogrammétriques de l'objet sont effectuées chacune avec une vue différente ;
un traitement d'image des prises de vues est réalisée, dans lequel les dispositions caractéristiques des marques ponctuelles qui correspondent les unes aux autres dans les prises de vue sont d'abord associées entre elles et, à l'aide de cette association, les marques ponctuelles comprises dans les dispositions caractéristiques des marques ponctuelles, et qui correspondent les unes aux autres dans les prises de vues, sont associées les unes aux autres, et
à l'aide de l'association des marques ponctuelles, la forme spatiale de l'objet est déterminée à l'aide d'un procédé d'analyse photogrammétrique.

10. Procédé selon la revendication 9, dans lequel l'application des marques ponctuelles sur l'objet est réalisée de telle sorte qu'une enveloppe élastique, sur laquelle les marques ponctuelles sont appliquées, soit enfilée sur l'objet.

11. Procédé selon la revendication 9, dans lequel l'application des marques ponctuelles sur l'objet est réalisée de telle sorte que des films autocollants, sur lesquels les marques ponctuelles sont appliquées, soient collés sur la surface de l'objet.

12. Procédé selon la revendication 9, dans lequel l'application des marques ponctuelles sur l'objet est réalisée de telle sorte que celles-ci soient projetées sur la surface de l'objet.

13. Procédé selon l'une des revendications précédentes, dans lequel les marques ponctuelles ne sont pas codées.

14. Procédé selon l'une des revendications précédentes, dans lequel les marques ponctuelles sont constituées de croix.

15. Procédé selon l'une des revendications 1 à 13, dans lequel les marques ponctuelles sont constituées des points d'intersection d'un réseau.

16. Dispositif de détermination photogrammétrique de la forme spatiale d'un objet avec un système de prises de vues permettant de réaliser des prises de vues photogrammétriques de différentes vues d'un objet et un système de mesure, d'analyse des prises de vues et de détermination de la forme spatiale d'un objet, **caractérisé en ce que** le dispositif comprend en outre des marques ponctuelles et une ou plusieurs marques superficielles appliquées à la surface de l'objet, qui comportent chacune plusieurs marques ponctuelles, qui constituent l'arrière-plan des marques ponctuelles et dont la surface présente un aspect optique caractéristique.

17. Dispositif selon la revendication 16, qui comprend, en outre, une enveloppe élastique sur laquelle les marques ponctuelles et une ou plusieurs marques superficielles sont appliquées et qui est conçue de telle sorte qu'elle puisse être enfilée sur l'objet et s'adapte ainsi à la forme de l'objet.

18. Dispositif selon la revendication 16, qui comprend, en outre, un ou plusieurs films autocollants sur lesquels sont appliquées les marques ponctuelles et une ou plusieurs marques superficielles et qui sont conçus de telle sorte qu'ils soient étroitement liés à l'objet lorsqu'ils sont collés à la surface de celui-ci.

19. Dispositif selon la revendication 16, qui comprend, en outre, un système de projection conçu de telle sorte que les marques ponctuelles et une ou plusieurs marques superficielles puissent être projetées sur l'objet.

20. Dispositif de détermination photogrammétrique de la forme spatiale d'un objet avec un système de prises de vues permettant de réaliser des prises de vues photogrammétriques de différentes vues d'un objet et un système de mesure, d'analyse des prises de vues et de détermination de la forme spatiale d'un objet, **caractérisé en ce que** le dispositif comprend, en outre, des marques ponctuelles appliquées sur la surface d'un objet de telle sorte que des groupes de plusieurs marques ponctuelles soient formés, moyennant quoi les marques ponctuelles sont disposées dans un groupe les unes par rapport aux autres de telle sorte qu'une disposition caractéristique des marques ponctuelles soit obtenue.

21. Dispositif selon la revendication 20, qui comprend, en outre, une enveloppe élastique sur laquelle les marques ponctuelles sont appliquées et qui est conçue de telle sorte qu'elle puisse être enfilée sur l'objet et s'adapte ainsi à la forme de l'objet.

22. Dispositif selon la revendication 20, qui comprend, en outre, un ou plusieurs films autocollants sur lesquels sont appliquées les marques ponctuelles et qui sont conçus de telle sorte qu'ils soient étroitement liés à l'objet lorsqu'ils sont collés à la surface de celui-ci.

23. Dispositif selon la revendication 20, qui comprend, en outre, un système de projection conçu de telle sorte que les marques ponctuelles puissent ainsi être projetées sur l'objet.

24. Procédé ou dispositif selon l'une des revendications précédentes dans lequel l'objet est une partie d'un corps humain.
